# EUROPEAN PATENT SPECIFICATION

(11) **EP 1 847 234 B1**
(45) Date of publication and mention of the grant of the patent: **22.07.2009**
(21) Application number: 07007911.6
(22) Date of filing: 18.04.2007
(51) Int. Cl.: A61F 2/06

(54) **Device for controlling the positioning of a stent graft fenestration**
Vorrichtung zur Steuerung der Positionierung eines Stent-Fensters
Dispositif de contrôle de la mise en place d'une fenestration de greffe d'endoprothèse

(30) Priority: 18.04.2006 US 379115
(43) Date of publication of application: 24.10.2007
(73) Proprietor: Medtronic Vascular, Inc., Santa Rosa, CA 95403 (US)
(72) Inventor: Greenan, Trevor, Santa Rosa, CA 95405 (US)
(74) Representative: Zimmermann, Gerd Heinrich

(56) References cited:
- WO-A-03/065933
- WO-A-20/05034810
- WO-A-20/06036690
- US-A1- 2005 131 517

## Description

### FIELD OF THE INVENTION

The invention relates to a stent graft having at least one fenestration for placement proximate a branch vessel. More particularly, the invention relates to a device and method for controlling the positioning of a stent graft fenestration with respect to the branch vessel.

### BACKGROUND OF THE INVENTION

Stent grafts are often used for treatment of the vasculature in a body to bypass and repair a defect in the vasculature. For instance, a stent graft may be used to span an abdominal aortic aneurysm. In many cases such damaged or defected portions of the vasculature may include or be proximate to a branch vessel, such as a mesenteric artery or a renal artery. Repairing such a vessel without providing blood flow into the branch vessel can cause problems. As such, a stent graft having a fenestration in a side wall thereof is utilized, wherein the fenestration is positioned to align with the opening, or ostium, of the branch vessel after deployment of the stent graft. Another stent graft often referred to as a branch graft, can then be deployed through the fenestration into the branch vessel to provide a blood flow path to the branch vessel.

One issue that exists in such a procedure is how to accurately position a fenestration in relation to the branch vessel. If the position of a fenestration is offset with respect to a branch vessel when the stent graft is deployed, it may be difficult to deploy guidewires and catheters from the stent graft into the branch vessel to enable correct positioning of the branch vessel stent graft. Also if the fenestration is offset from the branch vessel and a stent graft is deployed into the branch vessel from a primary stent graft, the branch vessel stent graft may be kinked to such an extent that blood flow will not occur there through.

US2005/0131517 discloses a stent-graft for treating an aneurysm near a vascular bifurcation. The stent-graft comprises a primary graft material forming a central lumen and defining at least one side-wall opening, and a flexible fenestration assembly mounted around the side-wall opening to allow access to a branch vessel. The assembly comprises an outer ring and an inner ring joined by a flexible corrugated graft material that is neither formed by nor integral with the primary graft material.

W02006/036690 discloses another stent-graft assembly for treating a vascular bifurcation. The assembly comprises a primary graft defining a central lumen and a side-wall opening, a branch graft with a proximal end stitched around the side-wall opening of the primary graft, and a positioning catheter extending within the central lumen of the primary graft and releasably attached to the branch graft. The branch graft is fully everted so as to lie inside the central lumen of the primary graft during delivery.

W02003/065933 discloses yet another stent-graft assembly for repairing aneurysms at the vicinity of vascular bifurcations, comprising a primary graft defining a central lumen and a side-wall opening, a branch graft with a flange formed around its proximal end and bonded around the side-wall opening of the primary graft, and a positioning catheter extending within the central lumen of the primary graft and releasably attached to the branch graft.

Custom devices known in the art are one solution to this problem; a need exists for a less customized device and method that provides active control of a stent graft fenestration during positioning relative to a branch vessel, particularly after release, or at least partial release, of the stent graft from a delivery system.

### BRIEF SUMMARY OF THE INVENTION

In view of the above, a stent graft according to claims 1 and 10 is provided. Further aspects, features, details, and advantages of the present invention are apparent from the dependent claims, the specification, and the accompanying drawings.

Various embodiments according to the present invention are directed to apparatus and methods of using an integrated member pre-attached to a flexible, translatable, fenestration of a stent graft that may be used to guide a catheter into engagement with the fenestration to provide control thereof after the stent graft is, at least partially, released from a delivery system within a patient's vascular system. Once alignment of the stent graft fenestration with a branch vessel has been achieved, the integrated member may be detached from the fenestration and removed, along with the catheter, from the patient's vascular system.

An embodiment according to the present invention is a stent graft for repair of an aneurysm proximate a branch vessel that includes a primary graft having an anchoring device, a graft material forming a central lumen and at least one flexible, translatable fenestration in a side wall thereof. A fenestration positioning member extends within the stent graft central lumen and has a distal end removably attached to the stent graft proximate the at least one fenestration. In an embodiment, the fenestration positioning member includes a tubular member having a wire extending there through, wherein a sidewall of the tubular member includes an aperture through which a loop of the wire extends and removably attaches to the flexible, translatable fenestration. In another embodiment, the fenestration positioning member includes a tubular member having a suture extending there through, wherein a loop of the suture distally extends from a distal end of the tubular member and is removably attached to the flexible, translatable fenestration. In another embodiment, the stent graft includes two flexible, translatable fenestrations, each fenestration having an integrated fenestration positioning member removably attached thereto.

In another embodiment according to the present invention, a stent graft for repair of an aneurysm proximate a branch vessel includes a primary graft having an anchoring device, a graft material defining a central lumen and having at least one flexible, translatable branch graft fenestration formed in a side wall of the graft material. The flexible branch graft fenestration defining a branch lumen in fluid communication with the central lumen of the primary graft upon deployment within a branch vessel. A fenestration positioning member extends within the primary graft central lumen and has a distal end removably attached to the flexible branch graft proximate to the at least one fenestration. In an embodiment, the flexible branch graft fenestration is compressible along a longitudinal axis. In another embodiment, the stent graft includes two flexible branch graft fenestrations, each having a fenestration positioning member detachably coupled thereto.

In another embodiment, a flexible, translatable branch graft fenestration may be axially compressible by including pleats for flexing and articulating the branch graft relative to a longitudinal axis of a branch vessel and/or may diminish in diameter from a branch lumen inlet to a branch lumen outlet. In such an embodiment, a fenestration positioning member may be detachably coupled to the graft material of the branch graft fenestration for flexing, translating and/or deploying the branch graft within the branch vessel.

Another embodiment according to the present invention includes a method of positioning a stent graft fenestration with respect to a branch vessel. The method includes providing a stent graft having at least one flexible, translatable fenestration and a fenestration positioning member removably attached proximate the fenestration. The stent graft is advanced to a treatment site within a main vessel proximate the branch vessel, wherein at least the portion of the stent graft that includes the flexible fenestration is allowed to expand to thereby provide access to the fenestration and the fenestration positioning member. A catheter, for example, a steerable catheter, is then advanced over the fenestration positioning member into engagement with the flexible fenestration, wherein the catheter is used to manipulate, e.g., flex, translate and/or axially extend, the fenestration into alignment with an ostium of the branch vessel. An anchoring device of the stent graft is then released to substantially fix the position of the fenestration with respect to the ostium of the branch vessel.

A further embodiment according to the present invention is a method of positioning a stent graft having two flexible, translatable fenestrations, wherein each fenestration has a fenestration positioning member removably attached thereto. The method includes advancing a catheter, for example, a steerable catheter, over each of the fenestration positioning member into engagement with the respective flexible fenestration, wherein the catheters are used to manipulate, e.g., flex, translate and/or axially extend, each fenestration into alignment with an ostium of a respective branch vessel. An anchoring device of the stent graft is then released to substantially fix the position of the fenestrations with respect to the ostium of the respective branch vessel. In a further embodiment, the method includes using the catheters to deliver guidewires through the flexible fenestrations into the branch vessels prior to removing the fenestration positioning members and the catheters from the vasculature. In another embodiment, the guidewires may be used to position branch grafts into the branch vessels, wherein branch lumens of the branch grafts are in fluid communication with a central lumen of the (main body) stent graft.

Another embodiment according to the present invention includes a method of positioning a branch graft fenestration of a stent graft with a branch vessel. The method includes providing a stent graft having at least one flexible branch graft fenestration and a fenestration positioning member removably attached proximate the branch graft fenestration. The stent graft is advanced to a treatment site within a main vessel proximate the branch vessel, wherein at least the portion of the stent graft that includes the flexible branch graft fenestration is allowed to expand to thereby provide access to the branch graft fenestration and the fenestration positioning member. A catheter, for example, a steerable catheter, is then advanced over the fenestration positioning member into engagement with the flexible branch graft fenestration, wherein the catheter is used to manipulate, e.g., flex, translate and/or axially extend, the branch graft fenestration into alignment with an ostium of the branch vessel. An anchoring device of the stent graft is then released to substantially fix the position of the branch graft fenestration with respect to the ostium of the branch vessel. In a further embodiment, the method includes using the catheter to deliver a guidewire through the flexible branch graft fenestration into the branch vessel prior to removing the fenestration positioning member and the catheter from the vasculature. In another embodiment, the guidewire may be used to position a second branch graft, or a branch graft extender, through the branch graft fenestration into the branch vessel, such that a branch lumen of the second branch graft is in fluid communication with a lumen of the branch graft fenestration and a central lumen of the stent graft.

### BRIEF DESCRIPTION OF DRAWINGS

The foregoing and other features and advantages according to the invention will be apparent from the following description as illustrated in the accompanying drawings. The accompanying drawings, which are incorporated herein and form a part of the specification, further serve to explain the principles according to the invention. The drawings are not to scale.

FIG. 1 illustrates a stent graft in accordance with an embodiment of the present invention.

FIG. 2A illustrates a fenestration positioning member of FIG. 1 in accordance with an embodiment of the present invention.

FIG. 2B illustrates a fenestration positioning member of FIG. 1 in accordance with another embodiment of the present invention.

FIG. 3 is a partial cross-sectional view of a flexible fenestration in accordance with another embodiment of the present invention.

FIG. 4 is a partial cross-sectional view of a flexible fenestration in accordance with another embodiment of the present invention.

FIG. 5A is a partial cross-section of an embodiment according to the present invention having a flexible branch graft fenestration aligned with an ostium of a branch vessel.

FIG. 5B illustrates the flexible branch graft fenestration of FIG. 5A positioned within the branch vessel.

FIG. 6A is a partial cross-section of another embodiment according to the present invention having a flexible branch graft fenestration aligned with an ostium of a branch vessel.

FIG. 6B illustrates the branch graft fenestration of FIG. 6A positioned within the branch vessel.

FIG. 6C illustrates the branch graft fenestration of FIG. 6B with a stent delivery catheter positioned therein.

FIGS. 7-15 illustrate a method of positioning fenestrations of a stent graft with a respective branch vessel in accordance with an embodiment of the present invention.

### DETAILED DESCRIPTION

Specific embodiments are now described with reference to the figures, wherein like reference numbers indicate identical or functionally similar elements. The terms "distal" and "proximal" when used in the following description with respect to the catheter are a position or direction relative to the treating clinician. "Distal" or "distally" are a position distant from or in a direction away from the clinician. "Proximal" and "proximally" are a position near or in a direction toward the clinician. The terms "distal" and "proximal" when used for the implanted device are used with respect to the direction of blood flow from the heart, wherein the proximal end denotes the end nearer the heart.

The following detailed description is merely exemplary in nature. Although the description of embodiments in accordance with the invention are in the context of treatment of blood vessels, such as the renal arteries, the embodiments may also be used in other passageways where it is deemed useful. Furthermore, there is no intention to be bound by any expressed or implied theory presented in the preceding technical field, background, brief summary or the following detailed description.

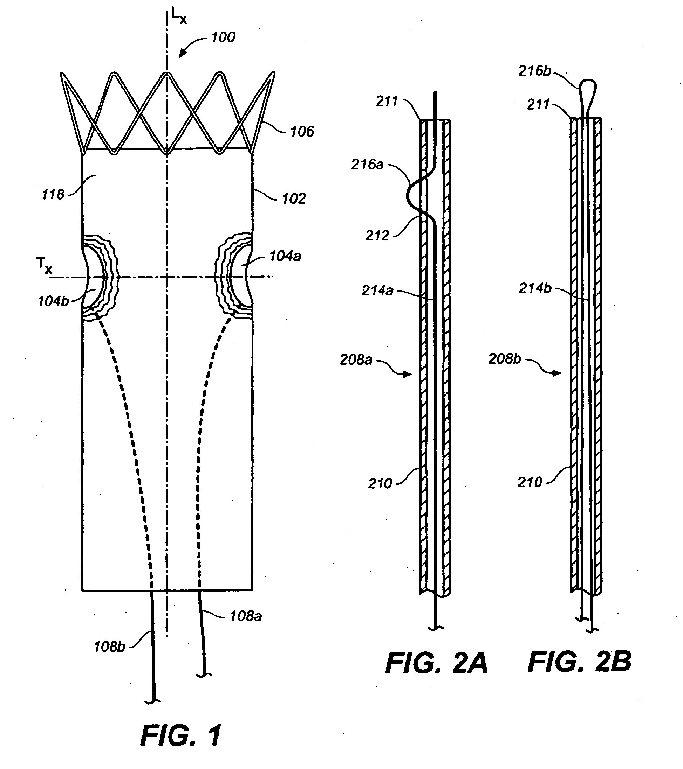

FIG. 1 illustrates a stent graft 100 that includes a primary graft 102 having two fenestrations, or openings, 104a, 104b. Accordingly, stent graft 100 may be used for repair of an aneurysm in a main vessel that is proximate two branch vessels, such as repair of an aneurysm in the aorta proximate the renal arteries. Various embodiments of stent graft 100 may include fewer or more fenestrations in accordance with the particular need of the patient.

As would be apparent to one of ordinary skill in the art, primary graft 102 includes a radially expandable reinforcement structure (not shown), which may include one or more stent-like structures, *e.g.,* Gianturco stents, attached to the graft material 118 forming a central lumen through stent graft 100. In various embodiments, graft material 118 may be comprised of woven polyester, polytetrafluoroethylene (ePTFE), and/or other biocompatible material. An anchoring device 106, which in the embodiment shown is a self-expanding bare spring, is attached at a distal end of primary graft 102 and is used to anchor primary graft 102 within the main vessel and to provide a substantially fluid-tight seal at the graft-vessel interface. In various embodiments, anchoring device 106 may be comprised of an anchoring structure, such as, a radially expandable stent, a frame, a series of rings, and/or adhesive, suture(s), staple(s), or other structures known for holding a stent graft in place.

In the embodiment shown in FIG. 1, primary graft 102 includes two flexible fenestrations 104a, 104b formed in or attached to a side wall of graft material 118. Flexible fenestrations 104a, 104b accommodate radial translation of the fenestrations with respect to a longitudinal axis Lₓ of primary graft 102 and upward, downward and sideward manipulation of the fenestrations with respect to a transverse axis Tₓ of primary graft 102. Described in another manner, flexible fenestrations 104a, 104b are independently manipulatable in an accordion-like manner to facilitate aligning each fenestration with an ostium of a respective branch vessel during deployment. Accordingly, flexible fenestrations 104a, 104b provide for re-positionable openings in primary graft 102 for access to the branch vessels, such that branch grafts may be deployed there through into the branch vessels. Although fenestrations 104a, 104b are shown roughly opposite one and other in primary graft 102, in various embodiments fewer or more fenestrations may be utilized and/or may be axially offset one from the other depending on the needs of the particular patient.

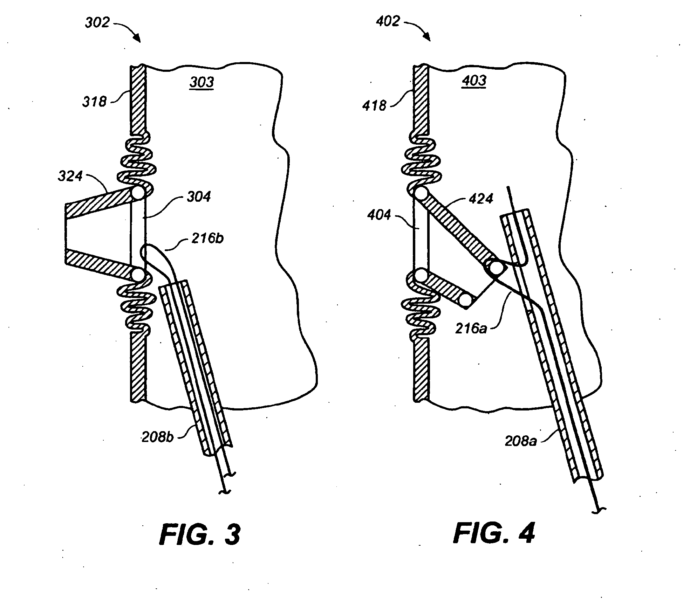

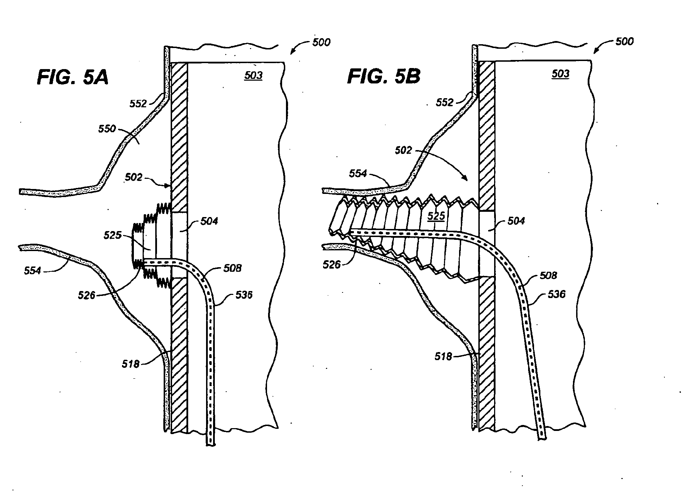

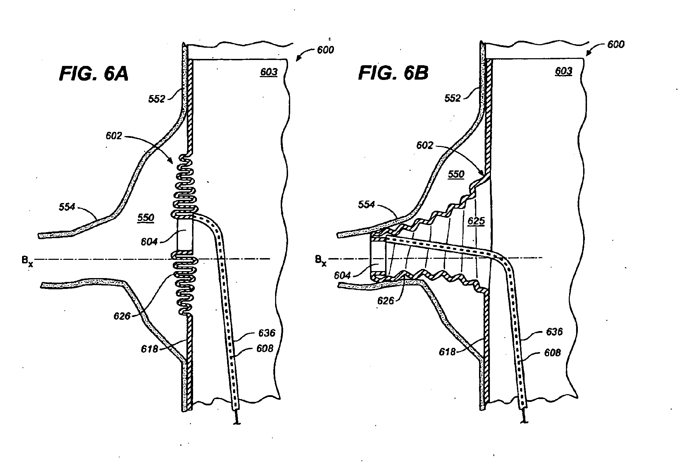

FIGS. 3 and 4 are partial cross-sectional views of a flexible fenestration in accordance with further embodiments of the present invention. Flexible fenestrations 304, 404 include a branch graft connector 324, 424, respectively, for delivering and/or coupling a branch graft thereto. Suitable branch graft connector assemblies that may be used in various embodiments of the present invention are disclosed in U.S. Patent No. 6,428,565 B1 and U.S. Pat. Appl. Pub. No. 2005/0131517 A1. Alternatively, as shown in the embodiments of FIGS. 5A and 6A, stent grafts 500, 600 include flexible, translatable branch graft fenestrations 526, 626 integrally extending from or coupled to a side wall of primary grafts 502, 602. Flexible branch graft fenestrations 526, 626 may be any of those disclosed in U.S. Appl. No. [to be assigned; Atty Dkt No. P1261 entitled Stent Graft Having A Flexible, Articulable, And Axially Compressible Branch Graft] filed on a date even herewith. The embodiments of FIGS. 3, 4, 5A and 6A are described in more detail below.

In the embodiment shown in FIG. 1, fenestration positioning members 108a, 108b extend within the central lumen of stent graft 100 from respective fenestrations 104a, 104b. Fenestration positioning members 108a, 1086 have a distal end removably attached to stent graft 100 proximate fenestrations 104a, 104b and are of a length, such that a proximal end (not shown) is accessible to the clinician outside the patient as stent graft 100 is delivered and positioned within a patient's vascular system. After at least partial release of stent graft 100 within a patient's vascular system, integrated fenestration positioning members 108a, 108b are used to guide a catheter, such as a steerable catheter 536 shown in FIGS. 5A and 5B, into engagement with fenestrations 104a, 104b to thereby aid in active control of each fenestration during positioning of the fenestration into alignment with an ostium of a respective branch vessel.

FIG. 2A illustrates a fenestration positioning member 208a that includes a tubular member 210 having a wire 214a extending there through. A sidewall of tubular member 210 includes an aperture 212 through which a loop 216A of wire 214a extends. In an embodiment, loop 216a passes through graft material 118 of stent graft 100, proximate to a fenestration, e.g., 104a, 104b, to removably or detachably couple fenestration positioning member 208a thereto. Fenestration positioning member 208a is released from stent graft 100 by withdrawing wire 214a proximally from tubular member 210 until wire 214a no longer engages stent graft 100.

In various other embodiments, loop 216a of fenestration positioning member 208a may be passed through, i.e., attached to, a stent graft reinforcement structure, a branch graft connector assembly and/or a flexible branch graft fenestration as may be apparent to one of ordinary skill in the art. Alternatively, rather than engaging loop 216a of fenestration positioning member 208a directly to the material or structure of stent graft 100, a piece of thread or suture material (not shown) may be sewed through both stent graft 100 and loop 216a of fenestration positioning member 208a and knotted, such that when fenestration positioning member 208a is released from stent graft 100, the thread or suture material remains with stent graft 100.

FIG. 2B illustrates a fenestration positioning member 208b in accordance with another embodiment of the present invention. Fenestration positioning member 208b includes a tubular member 210 having a length of thread or suture material 214b extending there through. Thread or suture 214b has a loop 216b that distally extends from a distal end 211 of tubular member 210. In an embodiment similar to the embodiment of FIG. 2A, loop 216b passes through graft material 118 of stent graft 100 proximate to fenestration 104 to removably couple fenestration positioning member 208b thereto. Fenestration positioning member 208b is released from stent graft 100 by proximally withdrawing thread or suture 214b from tubular member 210 until thread or suture 214b no longer engages stent graft 100.

Tubular member 210 may include a thin-walled, tubular structure of a metallic material, such as stainless steel, nitinol, or a cobalt-chromium superalloy. Such metallic tubing is commonly referred to as hypodermic tubing or a hypotube. Metallic tubing formed from other alloys, as disclosed in U.S. Patent No. 6,168,571, may also be used. In the alternative, tubular member 210 may include tubing made from a thermoplastic material, such as polyethylene block amide copolymer, polyvinyl chloride, polyethylene, polyethylene terephthalate, polyamide, or a thermoset polymer, such as polyimide.

FIG. 3 is a partial cross-sectional view of a flexible fenestration in accordance with another embodiment of the present invention. In this embodiment, primary graft 302 includes flexible fenestration 304 formed in a side wall of graft material 318 that has a branch graft connector portion 324 extending to an exterior thereof. Branch graft connector portion 324 may be used for receiving and coupling a branch graft (not shown) to primary graft 302. Suitable branch graft connector assemblies are disclosed in U.S. Patent No. 6,428,565 B1 and U.S. Pat. Appl. Pub. No. 2005/0131517 A1. Fenestration positioning member 208b extends proximally within central lumen 303 and is removably attached to flexible fenestration 304 by thread/suture loop 216b in a manner previously described. Alternatively, loop 216b could be coupled within or at an exterior end of branch graft connector portion 324. As in the previous embodiment, flexible fenestration 304 accommodates radial translation of the fenestration with respect to a longitudinal axis of primary graft 302 and upward, downward and sideward manipulation of the fenestration with respect to a transverse axis of primary graft 302 to thereby facilitate aligning branch graft connector portion 324 with an ostium of a respective branch vessel.

FIG. 4 is a partial cross-sectional view of a flexible fenestration in accordance with another embodiment of the present invention. In this embodiment, primary graft 402 includes flexible fenestration 404 formed in a side wall of graft material 418 that has a branch graft connector portion 424 extending within central lumen 403. Branch graft connector portion 424 may be used for receiving and coupling a branch graft (not shown) to primary graft 402. Suitable branch graft connector assemblies are disclosed in U.S. Pat. Appl. Pub. No. 2005/0131517 A1. Fenestration positioning member 208a extends proximally within central lumen 403 and is detachably coupled to branch graft connector portion 424 by wire 216a in a manner previously described. Alternatively, loop 216a could be coupled within branch graft connector portion 424 or at flexible fenestration 404. Flexible fenestration 404 provides "accordion-like" movement, as more fully described in the previous embodiments, to facilitate aligning branch graft connector portion 424 with an ostium of a respective branch vessel.

FIG. 5A is a partial cross-section of an embodiment according to the present invention within a vessel system. Stent graft 500 includes a primary graft 502 and an axially compressed branch graft 526 integrally coupled thereto. For clarity, radially expandable reinforcement structure and graft anchoring devices are not shown in FIGS. 5A and 5B, but would be used in such an embodiment as would be apparent to one of ordinary skill in the art. Primary graft 502 includes graft material 518 that defines central lumen 503 and has a flexible branch graft fenestration 526 integrally coupled to an opening 504 in a side wall thereof. Branch graft fenestration 526 includes a branch lumen 525 in fluid communication with central lumen 503 of primary graft 502. Flexible branch graft fenestration 526 includes pleats for flexing and translating branch graft 526 relative to its longitudinal axis, wherein the pleats of branch graft fenestration 526 are cylindrically shaped and diminish in diameter from a branch lumen inlet to a branch lumen outlet. Other suitable flexible and articulable branch graft fenestrations are disclosed in co-pending U.S. Appl. No. [to be assigned; Atty Dkt No. P1261 entitled Stent Graft Having A Flexible, Articulable, And Axially Compressible Branch Graft]. Similarly to the flexible fenestrations described in previous embodiments, flexible branch graft fenestration 526 accommodates radial translation of the fenestration with respect to a longitudinal axis of primary graft 502 and upward, downward and sideward manipulation of the branch graft fenestration with respect to a transverse axis of primary graft 502 to thereby facilitate aligning branch graft fenestration 526 with branch vessel 554.

In FIG. 5A, primary graft 502 is shown released within main vessel 552 for providing repair of aneurysm 550, which encompasses the ostium of branch vessel 554. Flexible branch graft fenestration 526 is shown slightly longitudinally offset from branch vessel 554. A fenestration positioning member 508 proximally extends within branch lumen 525 and central lumen 503 and has a distal end removably attached to branch graft fenestration 526 proximate opening 504.

FIG. 5B illustrates branch graft fenestration 526 of FIG. 5A bridging aneurysm 550 and laterally, upwardly extended into position within branch vessel 554. Branch graft 526 fenestration is laterally and upwardly extended by engaging catheter 536 with branch graft fenestration 526, as shown in FIG. 5A, and manipulating, *.i.e.,* flexing and/or torquing, catheter 536 distally until branch graft fenestration 526 is properly aligned with branch vessel 554. Once proper positioning of branch graft fenestration 526 is achieved, fenestration positioning member 508 and catheter 536 may be, consecutively or concurrently, removed.

FIG. 6A is a partial cross-section of another embodiment according to the present invention within a vessel system. Stent graft 600 includes a primary graft 602 comprised of graft material 618 that defines central lumen 603 and a flexible branch graft fenestration 626, which includes opening 604. In FIG. 2A, branch graft fenestration 626 is shown in an unexpanded, pre-deployment configuration, and, in this embodiment, is formed from graft material 618. Branch graft fenestration 626 is flexible and translatable to permit sideward and/or upward/downward manipulation for aligning opening 604 with an ostium of branch vessel 554.

In FIGS. 6A and 6B, primary graft 602 is shown released within main vessel 552 for providing repair of aneurysm 550, which encompasses the ostium of branch vessel 554, with opening 604 of flexible branch graft fenestration 626 longitudinally offset from branch vessel 554. Flexible branch graft fenestration 626 is laterally translatable along branch axis Bₓ, as well as upwardly, downwardly and sidewardly manipulatable with respect to branch axis Bₓ to enable subsequent alignment of the branch graft fenestration with branch vessel 554. A fenestration positioning member 608 proximally extends within central lumen 603 and has a distal end removably attached to a reinforcing ring (not shown) of opening 604 to control flexible branch graft fenestration 626 during alignment. Flexible branch graft fenestration 626 is sized to bridge aneurysm 550 in its deployed configuration.

FIG. 6B illustrates flexible branch graft fenestration 626 in its deployed configuration extended into branch vessel 554 to define a branch lumen 625 with opening 604 aligned with branch vessel 554. Proper alignment of opening 604 is accomplished through requisite manipulation of flexible branch graft fenestration 626 by catheter 636, which may include lateral translation along branch axis Bₓ, as well as upward, downward and/or sideward manipulation with respect to branch axis Bₓ.

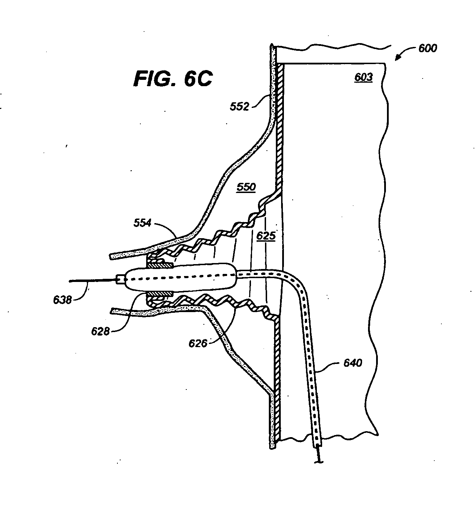

Once proper positioning of flexible branch graft fenestration 626 is achieved, catheter 636 may be used to deliver a guidewire 638 through opening 604. Fenestration positioning member 608 and catheter 636 may then be, consecutively or concurrently, removed. As illustrated in FIG. 6C, a stent delivery catheter 640 may then be tracked over guidewire 638 to deliver a branch lumen anchoring device 628 proximate opening 604, to thereby fix a position of branch graft fenestration 626 within branch vessel 554.

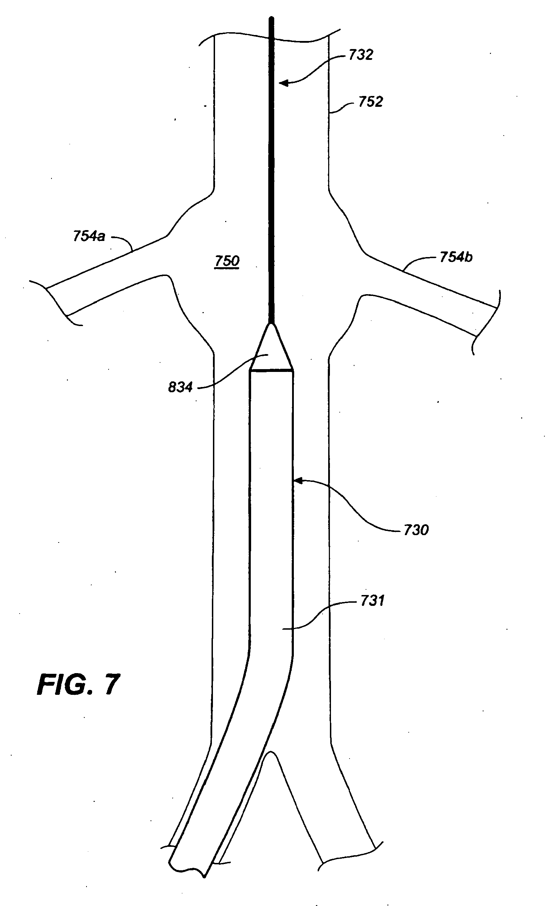

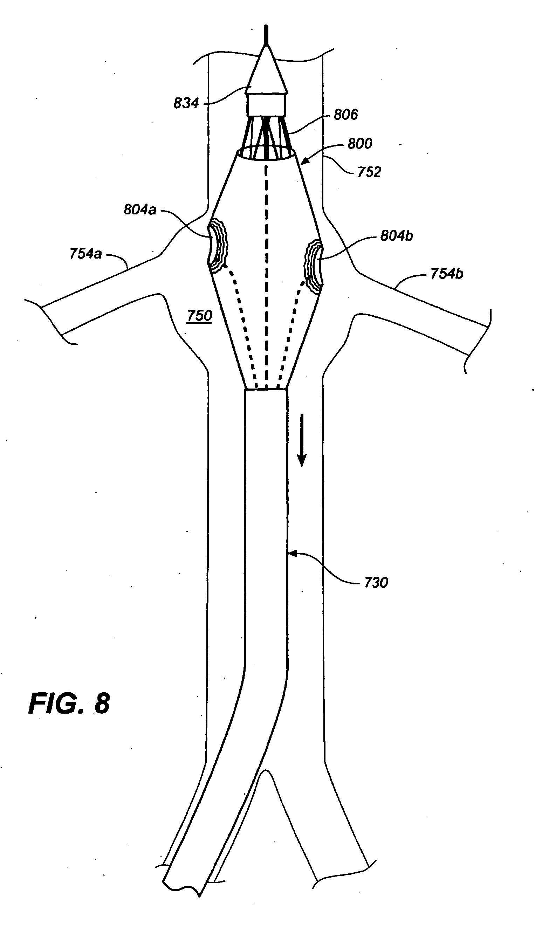

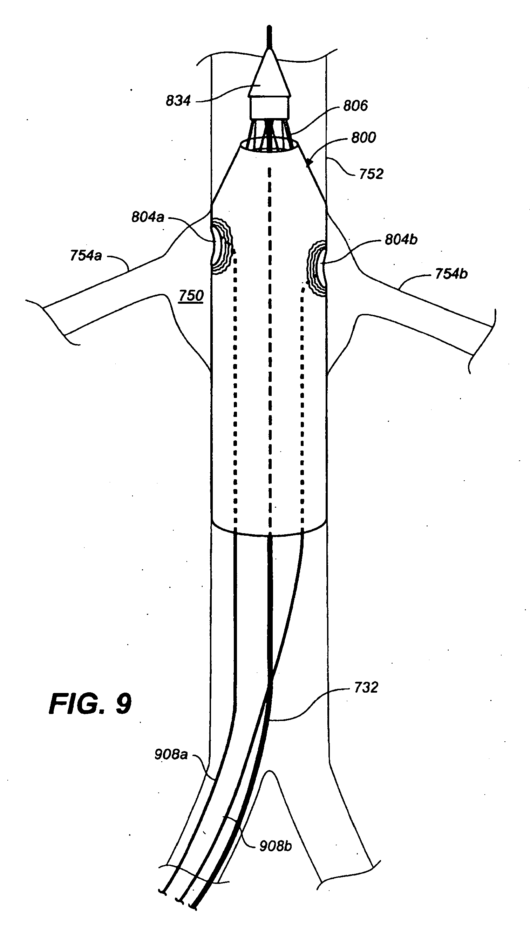

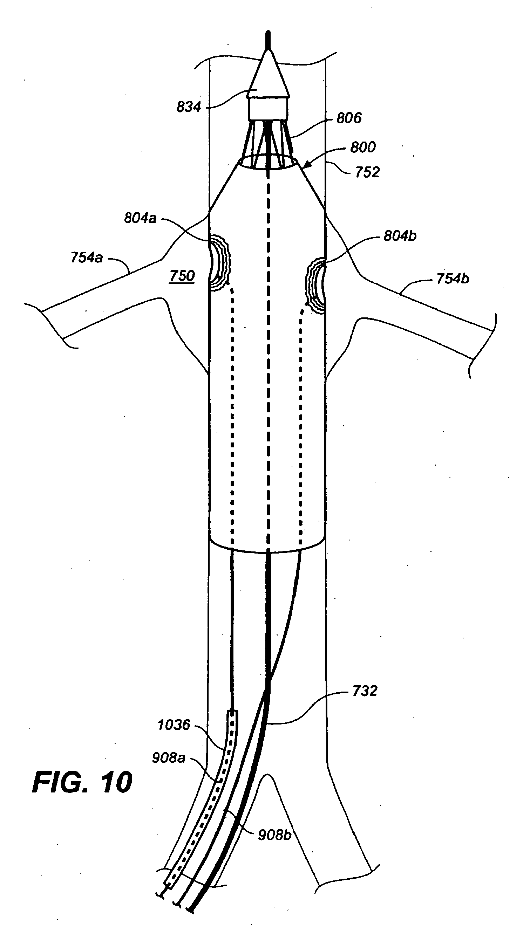

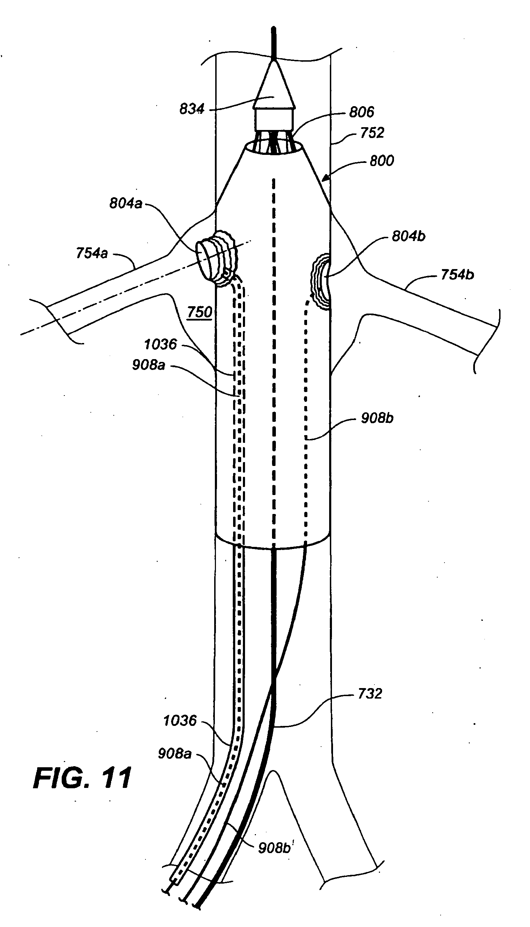

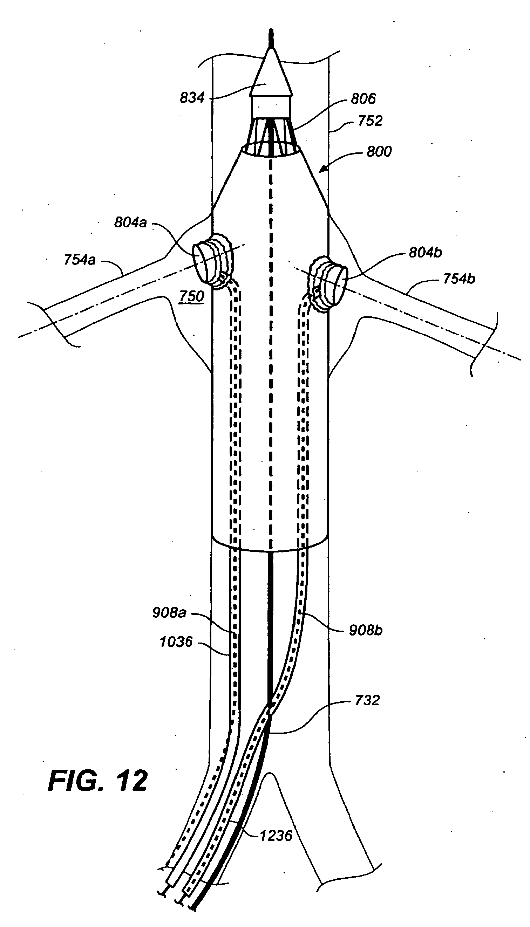

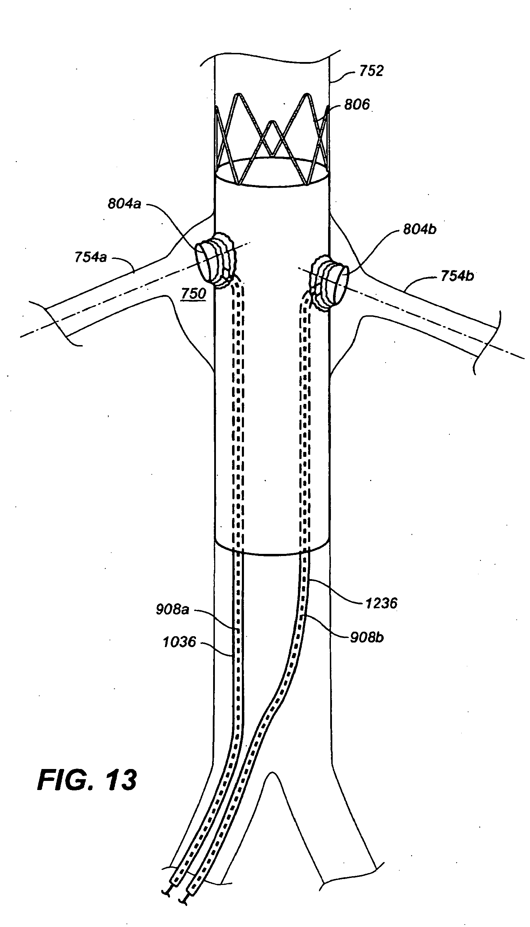

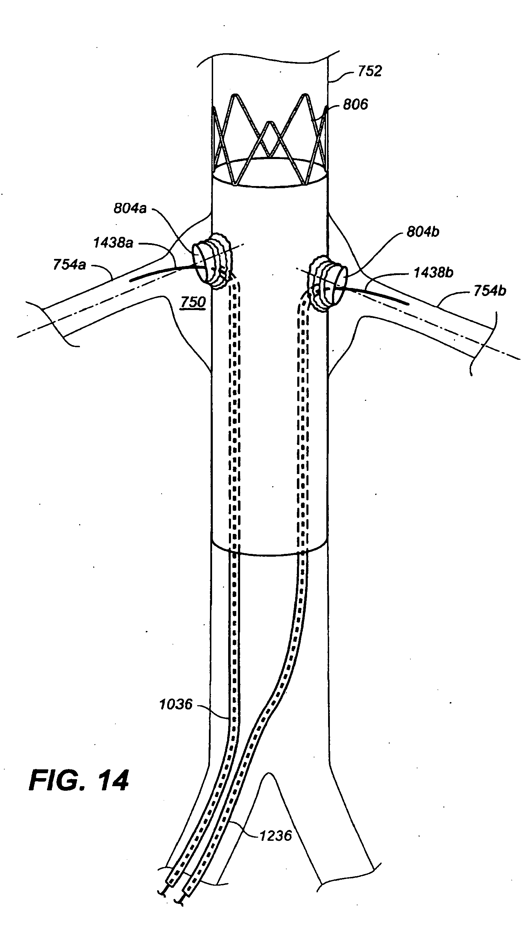

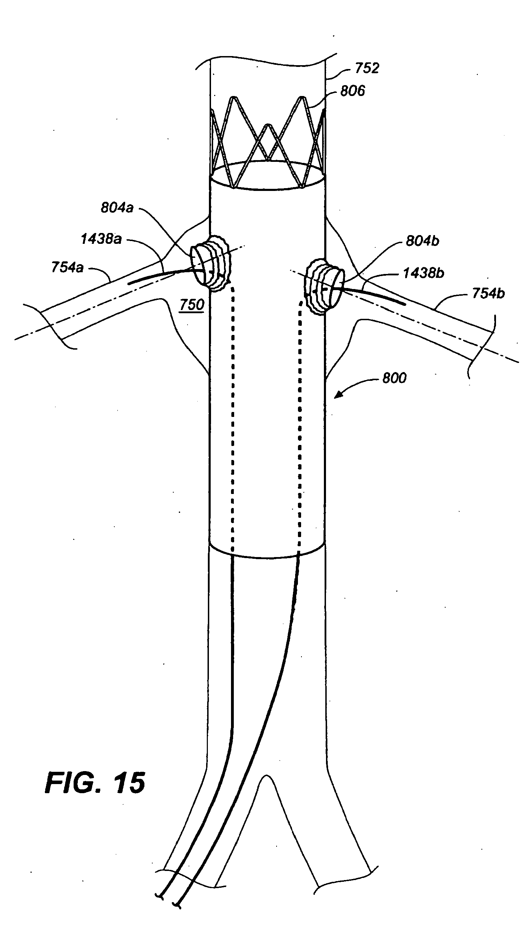

FIGS. 7-15 illustrate a method of positioning a fenestration of a stent graft with a branch vessel in accordance with an embodiment of the present invention. FIGS. 7-15 illustrate use of an embodiment according to the present invention in an aorta 752 for repair of an aortic aneurysm 750, which is situated at the ostiums of renal arteries 754a., 754b. A stent graft delivery catheter 730 having a sheath portion 731 and a guidewire portion 732 are advanced to a treatment site, *i.e.,* aneurysm 750, within aorta 752 proximate renal arteries 754a, 754b. A nosecone 834 of delivery catheter 730, which is attached to guidewire portion 732, is positioned proximal of aneurysm 750 and renal arteries 754a, 754b. Sheath portion 731 is then distally withdrawn thereby releasing at least a proximal portion of stent graft 800 to allow expansion of stent graft fenestrations 804a, 804b and to provide access to fenestration positioning members 908a, 908b. Suitable stent graft delivery catheters and methods of using are disclosed in U.S. Patent Appl. Publ. Nos. US2003/0233140 to Hartley et al. and US2004/0098079 to Hartley et al.

A first catheter 1036 is advanced over o fenestration positioning member 908a into engagement with flexible fenestration 804a. Catheter 1036 is then used to manipulate flexible fenestration 804 into alignment with the ostium of renal artery 754a. A second catheter 1236 is then advanced over the fenestration positioning member 908b into engagement with flexible fenestration 804b. Catheter 1236 is then used to manipulate flexible fenestration 804b into alignment with the ostium of renal artery 754b. In order to provide the required functionality, catheters 1036, 1236 should be steerable and flexible enough to readily advance through the patient's vasculature over fenestration positioning members 908a, 908b and also stiff enough to flex and torque fenestrations 804a, 804b into position with respective branch vessels 754a, 754b. A suitable catheter for this purpose is a Unison™ Steerable Sheath catheter manufactured by Enpath Medical, Inc. of Minneapolis, Minnesota. Thus, integrated fenestration positioning members 908a, 908b provide a pathway for catheters 1036, 1236 to allow for active control in positioning flexible fenestrations 804a, 804b after, at least partial, release of stent graft 800 within a main vessel. It would be apparent to one of ordinary skill in the art to provide radiopaque markers (not shown) around each flexible fenestration 804a, 804b to enable visualization by suitable radiographic techniques, which aids in proper positioning of fenestrations 804a, 804b with the respective branch vessel.

In an embodiment, anchoring device 806 of stent graft 800 may then be released from engagement with nosecone 834 to fully release primary stent 802 within aorta 752 and to fix the position of fenestrations 804a, 804b with respect to renal arteries 754a, 754b. Nosecone 834 with guidewire 732 may then be removed from the vessel system.

In another embodiment according to the present invention, prior to removal of fenestration positioning members 908a, 908b and catheters 1036, 1236, catheters 1036, 1236 may be used to deliver guidewires 1438a, 1438b through fenestrations 804a, 804b into renal arteries 754a, 754b. Guidewires 1438a, 1438b may then be used to position branch grafts [not shown] into renal arteries 754a, 754b, such that branch lumens of the branch grafts are in fluid communication with a central lumen of stent graft 800.

Methods of positioning and deploying one or more flexible branch graft fenestration devices, similar to or as shown in FIGS. 5A/5B and 6A/6B, and branch graft connector assemblies, similar to or as shown in FIGS. 3 and 4, can also be considered to be positioned in a manner similar to the method described. In various embodiments, an integrated fenestration positioning member of a stent graft according to the present invention is used to guide a steerable catheter into engagement with a flexible branch graft fenestration device or a branch graft connector assembly of the stent graft. After, at least partial, release of the stent graft within a main vessel, the fenestration positioning member allows for active control in positioning and/or deploying the device or assembly with respect to a branch vessel by manipulation of the catheter.

According to an embodiment of the present invention, a method of positioning a stent graft fenestration with respect to a branch vessel comprises providing a stent graft having at least one fenestration and an integrated fenestration positioning member removably attached proximate the fenestration; advancing the stent graft to a treatment site within a main vessel proximate the branch vessel; releasing at least a portion of the stent graft to allow expansion of the stent graft fenestration and to provide access to the fenestration positioning member; advancing a catheter over the fenestration positioning member into engagement with the fenestration; using the catheter to manipulate the fenestration into alignment with an ostium of the branch vessel; and releasing an anchoring device of the stent graft to fix the position of the fenestration with respect to the ostium of the branch vessel. According to one embodiment, the stent graft includes two fenestrations, each fenestration having a fenestration positioning member removably attached thereto. According to a further embodiment, the step of advancing a catheter includes advancing a catheter over each of the fenestration positioning member into engagement with the respective fenestration. Typically, the catheters can be used to deliver guidewires through the fenestrations into the branch vessels. Furthermore, the method may include removing the fenestration positioning members and the catheters. Also, the guidewires may be used to position branch grafts into the branch vessels, wherein branch lumens of the branch grafts are in fluid communication with a central lumen of the stent graft. In one embodiment of the method, the catheter is used to deliver a guidewire through the fenestration into the branch vessel. Furthermore, the method may include removing the fenestration positioning member and the catheter. According to another embodiment, the guidewire may be used to position a branch graft into the branch vessel, wherein a branch lumen of the branch graft is in fluid communication with a central lumen of the stent graft. Typically, the fenestration can be comprised of a flexible branch graft fenestration and wherein the fenestration positioning member is removably attached to the branch graft fenestration. In one embodiment of the method, the catheter is used for manipulating the flexible branch graft fenestration into alignment with the branch vessel.

It will be apparent to persons skilled in the relevant art that various changes in form and detail can be made therein without departing from scope of the invention. It will also be understood that each feature of each embodiment discussed herein, and of each reference cited herein, can be used in combination with the features of any other embodiment.

## Claims

1. A stent graft (100) for repair of an aneurysm proximate a branch vessel, the stent graft (100) comprising:
a primary graft (102) having an anchoring device (106), a graft material (118) forming a central lumen and at least one flexible fenestration (104a, 104b) in a side wall of the graft material (118); and
a fenestration positioning member (108a, 108b) extended within the central lumen, the fenestration positioning member (108a, 108b) having a distal end removably attached to the stent graft proximate the at least one flexible fenestration (104a, 104b).

2. The stent graft of claim 1, wherein the fenestration positioning member (208a) is comprised of a tubular member (210) having a wire extending (214a) therethrough.

3. The stent graft of claim 2, wherein a sidewall of the tubular member (210) includes an aperture (212) through which a loop (216a) of the wire (214a) extends and removably attaches to the fenestration (104a).

4. The stent graft of claim 1, wherein the fenestration positioning member (208b) is comprised of a tubular member (210) having a suture (214b) extending therethrough.

5. The stent graft of claim 4, wherein a loop (216b) of the suture (214b) distally extends from a distal end (211) of the tubular member (210) and is removably attached to the fenestration (104b).

6. The stent graft of claim 1, wherein the flexible fenestration (304) includes a branch graft connector portion (324) for guiding and receiving a branch graft therethrough.

7. The stent graft of claim 6, wherein the branch graft connector portion (324) extends from the fenestration (304) to an exterior of the primary graft (302).

8. The stent graft of claim 6 or 7, wherein the branch graft connector portion (324) extends from the fenestration (304) within the central lumen of the primary graft (302).

9. The stent graft of any of claims 6 to 8, wherein the fenestration positioning member (208b) is removably attached to the branch graft connector portion (324) of the fenestration (304).

10. A stent graft (500) for repair of an aneurysm proximate a branch vessel, the stent graft (500) comprising:
a primary graft (502) including an anchoring device, a graft material (518) defining a central lumen (503) and having at least one flexible branch graft fenestration (526) integral with a side wall of the graft material (518), wherein the branch graft fenestration (526) defines a branch lumen (525) in fluid communication with the central lumen (503) of the primary graft (502); and
a fenestration positioning member (508) extended within the central lumen (503), the fenestration positioning member (508) having a distal end removably attached to the flexible branch graft fenestration (526).

11. The stent graft of claim 10, wherein the branch graft fenestration (526) is compressible along a longitudinal axis.

12. The stent graft of claim 11, wherein the graft material forming the flexible branch graft fenestration (526) includes pleats for flexing and articulating the branch graft fenestration (526) relative to the longitudinal axis.

13. The stent graft of any of claims 10 to 12, wherein the pleats of the branch graft fenestration (526) are cylindrically shaped and uniform.

14. The stent graft of any of claims 10 to 12, wherein the pleats of the branch graft fenestration (526) are cylindrically shaped and diminish in diameter from a branch lumen inlet to a branch lumen outlet.

15. The stent graft of any of claims 10 to 14, wherein the flexible branch graft fenestration (526) is formed from the primary stent graft material (518) to be integral therewith.

16. The stent graft of any of claims 10 to 14, wherein the flexible branch graft fenestration (526) is integrally coupled to the primary stent graft material (518).

## Patentansprüche

1. Stent-Transplantat (100) zur Reparatur eines Aneurysmas nahe einem Zweiggefäß, wobei das Stent-Transplantat (100) umfasst:
ein primäres Transplantat (102) mit einer Verankerungsvorrichtung (106), einem Transplantatmaterial (118), das ein zentrales Lumen und mindestens ein flexibles Fenster (104a, 104b) in einer Seitenwand des Transplantatmaterials (118) bildet; und
ein Fensterpositionierungselement (108a, 108b), das sich innerhalb des zentralen Lumens erstreckt, wobei das Fensterpositionierungselement (108a, 108b) ein distales Ende aufweist, das entfernbar an dem Stent-Transplantat nahe dem mindestens einen flexiblen Fenster (104a, 104b) angeordnet ist.

2. Stent-Transplantat nach Anspruch 1, wobei das Fensterpositionierungselement (208a) ein rohrförmiges Element (210) mit einem Draht (214a), der sich durch dieses hindurch erstreckt, umfaßt.

3. Stent-Transplantat nach Anspruch 2, wobei eine Seitenwand des rohrförmigen Elements (210) eine Öffnung (212) enthält, durch die sich eine Schleife (216a) des Drahtes (214a) erstreckt und entfernbar an dem Fenster (104a) befestigt ist.

4. Stent-Transplantat nach Anspruch 1, wobei das Fensterpositionierungselement (208b) ein rohrförmiges Element (210) mit einem Nahtmaterial (214b), das sich durch dieses hindurch erstreckt, umfaßt.

5. Stent-Transplantat nach Anspruch 4, wobei eine Schleife (216b) des Nahtmaterials (214b) sich distal von einem distalen Ende (211) des rohrförmigen Elements (210) erstreckt und lösbar an dem Fenster (104b) befestigt ist.

6. Stent-Transplantat nach Anspruch 1, wobei das flexible Fenster (304) einen Zweigtransplantverbinderabschnitt (324) enthält, um ein Zweigtransplantat hindurch zu führen und aufzunehmen.

7. Stent-Transplantat nach Anspruch 6, wobei der Zweigtransplantverbinderabschnitt (324) sich von dem Fenster (304) in einen Außenraum des primären Transplantats (302) erstreckt.

8. Stent-Transplantat nach Anspruch 6 oder 7, wobei der Zweigtransplantverbinderabschnitt (324) sich von dem Fenster (304) innerhalb des zentralen Lumens des primären Transplantats (302) erstreckt.

9. Stent-Transplantat nach einem der Ansprüche 6 bis 8, wobei das Fensterpositionierungselement (208b) entfernbar an dem Zweigtransplantverbinderabschnitt (324) des Fensters (304) befestigt ist.

10. Stent-Transplantat (500) zur Reparatur eines Aneurysmas nahe einem Zweiggefäß, wobei das Stent-Transplantat (500) umfasst:
ein primäres Transplantat (502) mit einer Verankerungsvorrichtung, einem Transplantatmaterial (518), das ein zentrales Lumen (503) definiert und mindestens ein flexibles Zweigtransplantatfenster (526), das integral mit einer Seitenwand des Transplantatmaterials (518) ausgebildet ist, aufweist, wobei das Zweigtransplantatfenster (526) ein Zweiglumen (525) in Fluidverbindung mit dem zentralen Lumen (503) des primären Transplantats (502) definiert; und
ein Fensterpositionierungselement (508), das sich innerhalb des zentralen Lumens (503) erstreckt, wobei das Fensterpositionierungselement (508) ein distales Ende aufweist, das entfernbar an dem flexiblen Zweigtransplantatfenster (526) befestigt ist.

11. Stent-Transplantat nach Anspruch 10, wobei das Zweigtransplantatfenster (526) entlang einer Längsachse zusammenpressbar ist.

12. Stent-Transplantat nach Anspruch 11, wobei das Transplantatmaterial, das das flexible Zweigtransplantatfenster (526) bildet, Falten zum Biegen und Abwinkeln des Zweigtransplantatfensters (526) relativ zu der Längsachse enthält.

13. Stent-Transplantat nach einem der Ansprüche 10 bis 12, wobei die Falten des Zweigtransplantatfensters (526) zylindrisch geformt und gleichförmig sind.

14. Stent-Transplantat nach einem der Ansprüche 10 bis 12, wobei die Falten des Zweigtransplantatfensters (526) zylindrisch geformt und von abnehmendem Durchmesser von einem Zweiglumeneinlass zu einem Zweiglumenauslass sind.

15. Stent-Transplantat nach einem der Ansprüche 10 bis 14, wobei das flexible Zweigtransplantatfenster (526) aus einem primären Stent-Transplantatmaterial (518), einstückig mit diesem gebildet ist.

16. Stent-Transplantat nach einem der Ansprüche 10 bis 14, wobei das flexible Zweigtransplantatfenster (526) einstückig an das primäre Stent-Transplantatmaterial (518) gekoppelt ist.

## Revendications

1. Endoprothèse couverte (100) destinée à réparer un anévrisme à proximité d'une ramification d'un vaisseau, l'endoprothèse couverte (100) comprenant :
une greffe primaire (102) ayant un dispositif d'ancrage (106), un matériau de greffe (118) formant une lumière centrale et au moins une fenestration flexible (104a, 104b) dans une paroi latérale du matériau de greffe (118) ; et
un élément de positionnement de fenestration (108a, 108b) déployé à l'intérieur de la lumière centrale, l'élément de positionnement de fenestration (108a, 108b) ayant une extrémité distale fixée de manière amovible à l'endoprothèse couverte à proximité de la ou des fenestrations flexibles (104a, 104b).

2. Endoprothèse couverte selon la revendication 1, dans laquelle l'élément de positionnement de fenestration (208a) se compose d'un élément tubulaire (210) à l'intérieur duquel s'étend un fil métallique (214a).

3. Endoprothèse couverte selon la revendication 2, dans laquelle une paroi latérale de l'élément tubulaire (210) comprend une ouverture (212) à l'intérieur de laquelle s'étend une boucle (216a) du fil métallique (214a) qui se fixe à la fenestration (104a).

4. Endoprothèse couverte selon la revendication 1, dans laquelle l'élément de positionnement de fenestration (208b) se compose d'un élément tubulaire (210) à l'intérieur duquel s'étend une suture (214b).

5. Endoprothèse couverte selon la revendication 4, dans laquelle une boucle (216b) de la suture (214b) s'étend distalement d'une extrémité distale (211) de l'élément tubulaire (210) et se fixe de manière amovible à la fenestration (104b).

6. Endoprothèse couverte selon la revendication 1, dans laquelle la fenestration flexible (304) comprend une partie de connecteur de greffe ramifiée (324) destinée à guider et recevoir une greffe ramifiée à travers celle-ci.

7. Endoprothèse couverte selon la revendication 6, dans laquelle la partie de connecteur de greffe ramifiée (324) s'étend de la fenestration (304) à un extérieur de la greffe primaire (302).

8. Endoprothèse couverte selon la revendication 6 ou 7, dans laquelle la partie de connecteur de greffe ramifiée (324) s'étend de la fenestration (304) à l'intérieur de la lumière centrale de la greffe primaire (302).

9. Endoprothèse couverte selon l'une quelconque des revendications 6 à 8, dans laquelle l'élément de positionnement de fenestration (208b) est fixé de manière amovible à la partie de connecteur de greffe ramifiée (324) de la fenestration (304).

10. Endoprothèse couverte (500) destinée à réparer un anévrisme à proximité d'une ramification d'un vaisseau, l'endoprothèse couverte (500) comprenant :
une greffe primaire (502) comprenant un dispositif d'ancrage, un matériau de greffe (518) définissant une lumière centrale (503) et ayant au moins une
fenestration flexible pour greffe ramifiée (526)
faisant partie intégrante d'une paroi latérale du matériau de greffe (518), la fenestration pour greffe ramifiée (526) définissant une lumière ramifiée (525) en communication fluidique avec la lumière centrale (503) de la greffe primaire (502) ; et
un élément de positionnement de fenestration (508) s'étendant à l'intérieur de la lumière centrale (503),
l'élément de positionnement de fenestration (508)
ayant une extrémité distale fixée de manière amovible à la fenestration flexible pour greffe ramifiée (526).

11. Endoprothèse couverte selon la revendication 10, dans laquelle la fenestration pour greffe ramifiée (526) est compressible le long d'un axe longitudinal.

12. Endoprothèse couverte selon la revendication 11, dans laquelle le matériau de greffe formant la fenestration flexible pour greffe ramifiée (526) comprend des plis destinés à fléchir et articuler la fenestration pour greffe ramifiée (526) relativement à l'axe longitudinal.

13. Endoprothèse couverte selon l'une quelconque des revendications 10 à 12, dans laquelle les plis de la fenestration pour greffe ramifiée (526) sont cylindriques et uniformes.

14. Endoprothèse couverte selon l'une quelconque des revendications 10 à 12, dans laquelle les plis de la fenestration pour greffe ramifiée (526) sont cylindriques et ont un diamètre décroissant d'une entrée de lumière ramifiée à une sortie de lumière ramifiée.

15. Endoprothèse couverte selon l'une quelconque des revendications 10 à 14, dans laquelle la fenestration flexible pour greffe ramifiée (526) est formée à partir du matériau d'endoprothèse couverte primaire (518) de manière à faire partie intégrante de celle-ci.

16. Endoprothèse couverte selon l'une quelconque des revendications 10 à 14, dans laquelle la fenestration flexible pour greffe ramifiée (526) est intégralement couplée au matériau d'endoprothèse couverte primaire (518).
